# EUROPEAN PATENT SPECIFICATION

(11) **EP 4 119 169 B1**
(45) Date of publication and mention of the grant of the patent: **13.11.2024**
(21) Application number: 21186137.2
(22) Date of filing: 16.07.2021
(51) Int. Cl.: A61L 27/46, A61L 27/48, A61L 27/50, A61L 27/54, A61L 27/56, A61L 27/58

(54) **A CONSTRUCT FOR ARTICULAR CARTILAGE REGENERATION AND A METHOD OF PREPARATION**
KONSTRUKTION FÜR GELENKKNORPELREGENERATION UND VERFAHREN ZUR HERSTELLUNG
CONSTRUCTION POUR LA RÉGÉNÉRATION DU CARTILAGE ARTICULAIRE ET SON PROCÉDÉ DE PRÉPARATION

(43) Date of publication of application: 18.01.2023
(73) Proprietor: Lietuvos sveikatos mokslu universitetas, 44307 Kaunas (LT); Kauno Technologijos Universitetas, 44249 Kaunas (LT)
(72) Inventor: Usas, Arvydas, Kaunas (LT); Maciulaitis, Justinas, Kaunas (LT); Jankauskaite , Lina, Kaunas (LT); Griniute-Aukstikalne , Lauryna, Kaunas (LT); Pockevicius, Alius, Kaunas (LT); Dabasinskaite , Lauryna, Kaunas (LT); Martuzevicius, Dainius, Kaunas (LT); Malinauskas, Mantas, Kaunas (LT); Mickevicius, Tomas, Kaunas (LT); Rimkunas, Augustinas, Kaunas (LT); Baniukaitiene, Odeta, Kaunas (LT); Krugly, Edvinas, Kaunas (LT); Ciuzas, Darius, Kaunas (LT)
(74) Representative: Zaboliene, Reda

(56) References cited:
- JP-A- 2004 194 944
- US-A1- 2011 250 689
- US-A1- 2013 338 791
- US-A1- 2019 175 786
- FORMICA FLORIAN A. ET AL: "A Bioinspired Ultraporous Nanofiber-Hydrogel Mimic of the Cartilage Extracellular Matrix", ADVANCED HEALTHCARE MATERIALS, vol. 5, no. 24, 25 November 2016 (2016-11-25), DE, pages 3129 - 3138, XP055873444, ISSN: 2192-2640, Retrieved from the Internet <URL:https://api.wiley.com/onlinelibrary/tdm/v1/articles/10.1002%2Fadhm.201600867> DOI: 10.1002/adhm.201600867

## Description

### FIELD OF THE INVENTION

The present invention relates to implantable polymeric construct for regeneration of articular cartilage (further referred to as regeneration construct) damaged by trauma or chronic inflammatory-degenerative process. The present invention relates to a method of producing an articular cartilage.

### DESCRIPTION OF THE RELATED ART

Articular surfaces of the joints, such as knee or shoulder joint, are covered with cartilage tissue to prevent direct friction of the bones that make up the joint. A damage of the cartilage tissue due to aging, heavy or repetitive overloads lead to inflammation of the joint, which causes pain and movement disorders and gradual development of osteoathritis. In fact, articular cartilage tissue damage is often found in both young and elderly patients. In order to alleviate the symptoms and improve quality of patient's life there is growing demand for more efficient methods to restore damaged articular cartilage which has no intrinsic capacity to regenerate on its own. The biggest challenge in the treatment of damaged articular cartilage is to develop the optimal method of cartilage repair, capable of regenerating long lasting hyaline cartilage.

Due to the increased clinical need to restore articular cartilage, improved tissue repair techniques and new tissue engineering products continue to be developed. These products are mainly related to the use of biomaterials for the development of osteochondral implants to restore anatomically the bilayer structure of natural osteochondral tissue while maintaining mechanical properties of repair tissues. KR20140098085 relates to an adhesion preventing layer which can prevent abnormal tissue adhesion and, more specifically, to an adhesion preventing layer having a multi-layered nanofibrous structure manufactured by an electrospinning method and containing a hydrophilic natural polymer such as chitosan and the like and a manufacturing method thereof. The manufacturing method is comprised of: electrospinning a mixed polymer solution including a biodegradable natural polymer to form a hydrophilic substrate layer on non-woven fabric; and electrospinning a mixed polymer solution including a hydrophobic biodegradable synthetic polymer on top thereof to form a hydrophobic layer on the non-woven fabric, thereby allowing the hydrophobic layer to inhibit cell proliferation toward the wound portion in an organ adjacent to the abdominal wall while a wound is healed by the hydrophilic layer and keep playing a role of a physical barrier until the wound is healed.

CN201810102243 belongs to the field of a biomaterial, and more specifically relates to an oriented porous composite electrospinning fiber scaffold having a bionic surface. The oriented porous composite electrospinning fiber scaffold is formed by composite fibers with single oriented arrangement and having a surface distributed with nano pores. The composite fibers takes the L-polylactide electrospinning fiber with the single oriented arrangement and having the surface distributed with nano pores as a matrix. The surface of the matrix is modified with polydopamine, or the matrix surface modified with polydopamine is grafted with a cartilage restoration-promoting drug.

US2013338791 provides a synthetic anisotropic, layered construct which exhibits zonal organisation and can be used to assist with cartilage formation. The invention also provides an electrospinning process for producing the construct. The construct mimics articular cartilage in terms of fibre organization and mechanical properties and is comprised of at least three distinctly different fibre layers in terms of i) fibre size (i.e. diameter of the fibre), ii) fibre organization or alignment, iii) fibre mechanics in terms of elongation, tensile modulus, and ultimate tensile strength. Specifically, this design is focused on exhibiting decreasing tensile strength, decreasing tensile modulus, with varying fibre organization. As well as exhibiting mechanical properties (compressive and tensile) suitable to provide mechanical integrity of native cartilage, the layered construct of the invention provides a scaffold through which chondrocytes or stem cells can penetrate and remain viable.

US201213472173 described a cartilage which is constructed using biodegradable electrospun polymeric scaffolds seeded with chondrocytes or adult mesenchymal stem cells. More particularly engineered cartilage has been prepared where the cartilage has a biodegradable and biocompatible nanofibrous polymer support prepared by electrospinning and a plurality of chondrocytes or mesenchymal stem cells dispersed in the pores of the support. The tissue engineered cartilages of the invention possess compressive strength properties similar to natural cartilage. Methods of preparing engineered tissues, including tissue engineered cartilages, are provided in which an electrospun nanofibrous polymer support is provided, the support is treated with a cell solution and the polymer-cell mixture cultured in a rotating bioreactor to generate the cartilage. The invention provides for the use of the tissue engineered cartilages in the treatment of cartilage degenerative diseases, reconstructive surgery, and cosmetic surgery. WO2019018443 provides a device for regenerating musculoskeletal tissue having a scaffold comprised of fiber layers adapted to provide mechanical integrity to the scaffold in the form of increased tensile and compressive resistance and one or more other layers adapted to provide mechanical integrity and to provide a suitable biochemical environment.

US 2019/175786 A1 discloses methods of using electrospun fibers to repair and/or regrow hyaline cartilage, e.g. on the articulating surfaces of bones.

### SUMMARY OF THE INVENTION

The invention relates to a method as disclosed in claim 1. The base of the regeneration construct scaffold is a biodegradable polymer. The biodegradable polymer is blended with natural additives, such as cellulose and hydroxyapatite which increase tissue affinity. The regeneration construct is composed of two layers, where layer I comprises blend of biodegradable polymer and cellulose and is prepared for cartilage tissue formation; and layer II comprises blend of biodegradable polymer, cellulose, and hydroxyapatite and is prepared for subchondral bone tissue formation. The construct has a fibrous morphology that accurately replicates the functions and structure of the natural cartilage. The regeneration construct is composed of polymer fibres providing inner space for deposition of cartilaginous extracellular matrix. The surface of the fibres is chemically modified. A biologically active component, growth factor, incorporated into the construct facilitates the migration, proliferation and differentiation of cells and production of extracellular matrix. After implantation of the regeneration construct, the active components are released over time, resulting in the regeneration of cartilage-like tissue through interactions with host and transplanted stem cells.

A construct for regeneration of articular joint cartilage comprises two-layer scaffold matrix, in which a layer I comprises an ozone-treated scaffold of biodegradable polymers and natural additives; and a layer II comprises a scaffold of biodegradable polymer and natural additives; and the layer I contains embedded biologically active molecules that are slowly and continuously released during operation of the construct.

The layer I and the layer II are made of a biodegradable polymer optionally selected from polycaprolactone, polylactic acid, poly(lactic-co-glycolic acid).

The natural additive of the layer I is cellulose and the natural additive of the layer II is cellulose and hydroxyapatite.

The biologically active molecules encapsulated in the layer I are growth factors which are incorporated to the biodegradable scaffold through functional groups, after the implantation the scaffold starts to degrade, this way releasing the growth factors in a controlled and sustained manner.

The biodegradable polymer is a porous material with a pore size in the range of 150 to 450 µm, preferably 200 to 400 µm.

The surface of ozone treated regeneration construct layer I scaffold is hydrophilic, with average water contact angle in range from 60 to 80°. The regeneration construct layer II scaffold is super hydrophilic, the water contact angle is 0°.

According to the invention, a method of preparation of the regeneration construct is provided, comprising preparation of polymer solutions for layers I and II; preparation of the regeneration construct layer I and II scaffolds by solution cryo-electrospinning; conversion of cellulose acetate to cellulose of the regeneration construct layer I and II scaffolds; surface modification of the regeneration construct layer I scaffold; embedding of biologically active molecules in the regeneration construct layer I scaffold; sterilization of the regeneration construct layer I and II scaffolds; combining of the regeneration construct layer I and II scaffolds in order to form final regeneration construct.

The method of preparation of the regeneration construct additionally comprises drying steps following to (b), (d) and (e) steps.

The biologically active molecules added to the solution of the first layer are growth factors which are bound only on fibers of the regeneration construct layer I scaffold surface. The growth factors are incorporated to the biodegradable scaffold through functional groups. The growth factors stimulate differentiation of stem cells to form chondral or subchondral tissue of layer I and layer II accordingly.

The solvents used for the preparation of polymer blend solutions are polar organic solvents.

The regeneration construct is for use in regenerating cartilage by implantation in a subject in need thereof.

After the implantation of the regeneration cartilage the scaffold starts to degrade, this way releasing the growth factors in a controlled and sustained manner. When released, growth factors stimulate differentiation of stem cells to form chondral or subchondral tissue accordingly. The latter is also stimulated by hydroxyapatite, which is the addition of regeneration construct layer II. After implantation and gradual degradation, regeneration construct withdraws from the defect site, making space for newly formed cartilage and subchondral tissue.

The advantage of the invention in comparison with the already developed methods is a unique sequence of multiple stages of production resulting in the regeneration construct with unique properties.

### BRIEF DESCRIPTION OF DRAWINGS

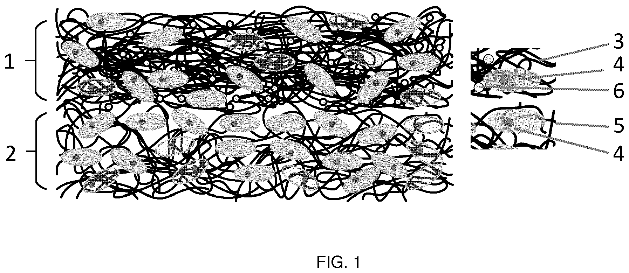

Fig. 1. Structure of the regeneration construct. 1 - regeneration construct layer I, 2 - regeneration construct layer II, 3 - fibrous scaffold of regeneration construct layer I, 4 - stem cells, 5 - fibrous scaffold of regeneration construct layer II, 6 - growth factor.
Fig. 2. Stages of regeneration construct fabrication
Fig. 3. Release kinetics of growth factor

### DETAILED DESCRIPTION OF THE INVENTION

The articular cartilage regeneration construct is composed of two layers, where a mixture of synthetic and natural polymers (PCL and cellulose) is used to produce the first layer (chondral layer) and the mixture of synthetic and natural polymer (PCL and cellulose) and hydroxyapatite (HAP) as bioactive substance, which is naturally formed in the bone structure is used for the production of the second layer (subchondral layer).

The preparation of the regeneration construct layer I comprises following stages:
1. Preparation of polymer blend solution by mixing synthetic and natural polymers and cellulose;
2. Cryo-electrospinning of solution of stage 1;
3. Drying of the regeneration construct layer I scaffold;
4. Conversion I cellulose acetate to cellulose of layer;
5. Surface modification of regeneration construct layer I scaffold;
6. Drying of regeneration construct layer I scaffold;
7. Binding of growth factor to the regeneration construct layer I scaffold;
8. Drying of regeneration construct layer I scaffold by lyophilization;
9. Sterilization of regeneration construct layer I scaffold.

The preparation of the regeneration construct layer II comprises following stages:
1. Preparation of polymer blend solution by mixing synthetic and natural polymers, cellulose and hydroxyapatite (HAP);
2. Cryo-electrospinning of solution of stage 1;
3. Drying of regeneration construct layer II scaffold;
4. Conversion cellulose acetate to cellulose of layer II;
5. Drying of regeneration construct layer II scaffold;
6. Sterilization of regeneration construct layer II scaffold.

The final regeneration construct is achieved by combining layer I and layer II in order so that layer I is positioned on top of layer II.

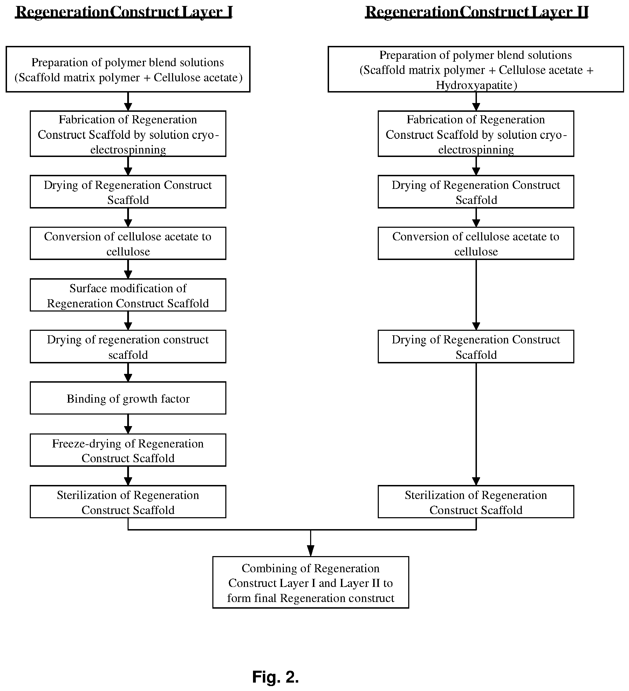

Stages of the method of preparation of regeneration construct are schematically presented in **Fig. 2****.**

### The method of preparation of the regeneration construct

### 1. Preparation of polymer blend solutions for layers I and II

The polymer solutions for regeneration construct layer I scaffold are being prepared by dissolving scaffold polymer (either polycaprolactone (IUPAC: (1,7)-Polyoxepan-2-one, CAS: 24980-41-4) or polylactic acid (IUPAC: poly(2-hydroxypropanoate), CAS: 26100-51-6), or poly(lactic-co-glycolic acid) (IUPAC: 2-(2-hydroxyacetyl)oxypropanoic acid, CAS 26780-50-7) and cellulose acetate (IUPAC name: [(2R,3S,4S,5R,6R)-5-acetyloxy-3,4,6-trihydroxyoxan-2-yl]methyl acetate CAS: 9004-35-7) by weight ratio of 2:1, in solvent mixture of acetone (IUPAC name: propan-2-one, CAS: 67-64-1) and N,N-dimethylformamide (IUPAC name N,N-Dimethylmethanamide, CAS: 68-12-2) by volumetric ratio of 2:3 to obtain polymer solution concentration from 10 to 40 % (by weight to volume ratio). The preparation of polymers solution is carried out at temperature range from 30 to 50 °C, by stirring for 3 to 10 hours.

The polymer solutions for the regeneration construct layer II scaffold are prepared by dissolving either polycaprolactone or polylactic acid or poly(lactic-co-glycolic acid) and cellulose acetate by weight ratio of 2:1, in mixture of acetone and N,N-dimethylformamide by volumetric ratio of 2:3, to obtain polymer solution of concentration from 10 to 40 % (by weight to volume). The preparation of polymer solution is carried out at temperature from 30 to 50 °C, by stirring for 3 to 10 hours. Hydroxyapatite (IUPAC name Pentacalcium hydroxide triphosphate, CAS:12167-74-7) is added to the final mixture to obtain concentration from 1 to 15 % and then continuously stirred at temperature from 30 to 50 °C, for 1 to 15 additional hours to obtain homogeneous solution.

### 2. Preparation of regeneration construct layer I and II scaffolds by solution cryo-electrospinning

The polymer blend solutions are loaded into a polymer blend solution reservoir. The polymer blend solution is supplied by constant flow rate pump. The polymer solution is supplied continuously into the spinning head at flow rate from 0.5 to 10.0 mL/h per spinning head for fabrication of regeneration construct layer I scaffold and at flow rate from 0.25 to 8.0 mL/h per spinning head for preparation of regeneration construct layer II scaffold. Between the spinning head and collector, high voltage is applied to facilitate electrospinning process. During electrospinning process acetone and N, N-dimethylformamide evaporate from polymer fibbers. Grounded rotating drum collector is used for collection of electrospun polymer structure. The temperature of the collector is kept from -10 to -40 °C. The distance from spinning head and the collector is ranging from 5 to 25 cm. High voltage is modulated by a high voltage supply up to 10-35 kV. The temperature of reservoir of polymer blend solution and the supply lines is kept in range from 30 to 60°C. The fabrication environment is maintained within the range of 20-40 °C for temperature and 20-40 % for relative humidity.

### 3. Drying of regeneration construct layer I and II scaffolds

After electrospinning, both regeneration construct layer I scaffolds and regeneration construct layer II scaffolds are placed in a vacuum chamber from 1 × 10⁻⁶ to 1 × 10⁻⁴ mPa at the temperature of 30 °C for duration of 12 to 72 h.

### 4. Conversion of cellulose acetate to cellulose of regeneration construct layer I and II scaffolds

The regeneration construct layer I scaffold and regeneration construct layer II scaffold are further immersed in 0.01 to 0.1 M NaOH (CAS: 1310-73-2)/water-ethanol (CAS: 64-17-5) solution at temperature from 15 to 25 ºC for 12-36 hours. Ethanol concentration is in range from 1 to 20 vol. %. After conversion samples are thoroughly rinsed with distilled water until pH value becomes neutral.

### 5. Surface modification of regeneration construct layer I scaffold

The regeneration construct layer I scaffold is immersed in deionized water and treated with ozone, which is bubbled from ozone generator. Ozone generator has an output range from 50 to 500 mg/h. The treatment is performed at temperature range from 15 to 30 °C for the period 10 to 60 min.

### 6. Drying of regeneration construct layer I and II scaffolds

After surface modificationthe regeneration construct layer I scaffold is placed in a vacuum chamber from 1 × 10⁻⁶ to 1 × 10⁻⁴ mPa at the temperature of 30 °C for duration of 12 to 72 h.

### 7. Binding of growth factor to the regeneration construct layer I scaffold

The growth factors are bound only on fibers of the regeneration construct layer I scaffold surface. The growth factors that could be used are: Transforming growth factor beta-3 (TGFβ-3), Insulin-like growth factor-1 (IGF-1), or Bone morphogenetic protein-7 (BMP-7)). The vial with the growth factor is carefully centrifuged prior to opening and then reconstituted accordingly to the manufacturer's instructions. Direct incorporation is subsequently used to immobilize growth factor per 4 to 6 mm diameter of the regeneration construct layer I scaffold.

### 8. Freeze-drying of the regeneration construct layer I scaffold

The regeneration construct layer I scaffold is frozen at temperature range from -10 to - 30 °C for duration of 3 to 15 h, and then freeze dried in vacuum from 1 × 10⁻⁶ to 1 × 10⁻⁴ mPa at the temperature of -20 to -50 °C for duration of 12 to 72 h.

### 9. Sterilization of the regeneration construct layer I and II scaffolds

Sterilization of the regeneration construct layer I scaffold and regeneration construct layer II scaffold is initiated with a preconditioning of the samples and is carried out at temperature range from 25 to 55°C for duration of 5 to 15 hours at relative humidity range from 30 to 60 %. The sterilization performed by 100% ethylene oxide (IUPC: oxirane) environment at temperature range from 40 to 60 °C for the duration of 100-200 minutes.

### The preparation of the regeneration construct scaffold for implantation

A method of preparation of the regeneration construct comprises preparation of layer I and layer II and combining layer I and layer II in order so that layer I is positioned on top of layer II.

### Properties of the prepared regeneration construct scaffold

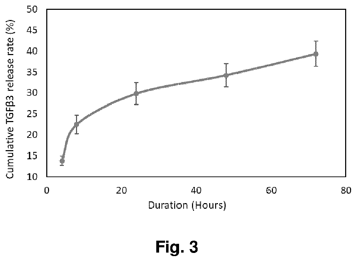

The regeneration construct scaffold has randomly orientated fibers with interconnected pores. In the regeneration construct layer I scaffold fibers diameter ranges from 3.3 µm to 20.1 µm. Intra-fiber porosity is in range from 75 to 95 % by volume, the average pore diameter is in range from 50 to 250 µm. Young's modulus is in range from 1 to 4 MPa. After the cellulose acetate to cellulose conversion the broad absorption band in the 3700-3000 cm⁻¹ range appeared in FTIR spectra due to introduced hydroxyl groups in the polymer chains, confirming cellulose acetate conversion into cellulose. The surface of ozone treated regeneration construct layer I scaffold is hydrophilic, with average water contact angle in range from 60 to 80°. The absorption of 410 to 800 % by mass of Phosphate-Buffered Saline solution is obtained after ozone treatment. The melting point of the regeneration construct layer I scaffold is in range from 55 to 65 ºC. Growth factor is bound to the scaffold via functional groups. 20 to 200 ng of growth factor is separately loaded on the regeneration construct layer I scaffold and approximately from 20 to 60 % of growth factor is released to the culture medium within 72 hours after incorporation **(****Fig. 3****).**

The diameter of the regeneration construct layer II scaffold fibers is 4 to 25 µm. Porosity is 80 to 97 % by volume, with average pore diameter of range from 100 to 400 µm. Young's modulus is 1 to 5 MPa. Hydroxyapatite particles (average diameter range from 5 to 25 µm, 1-10% w/v) are distributed evenly all over the fiber volume. The FTIR absorption band in the regeneration construct layer II at 1035 cm⁻¹ corresponded to the POa group in hydroxyapatite, and the band at 3570 cm⁻¹ is assigned to the hydroxyl group present in hydroxyapatite. The regeneration construct layer II scaffold is super hydrophilic, the water contact angle is 0°. The regeneration construct layer II scaffold absorbs 400 to 700 % by mass of Phosphate-Buffered Saline solution. The melting peak of the regeneration construct layer II scaffold is in range from 55 to 65 ºC.

### Operating principle of the regeneration construct

The regeneration construct layer I is seeded with somatic muscle-derived stem cells to produce hyaline-like cartilage tissue formation under physiological conditions *in vitro* and after implantation into articular cartilage defect *in vivo.* The pores between the fibres of regeneration construct play an essential role, as they guarantee the interconnectivity along the scaffold surface, ensuring proper cell penetration, migration and adherence. Since growth factors are incorporated to the biodegradable scaffold through functional groups, after the implantation the scaffold starts to degrade, this way releasing the growth factors in a controlled and sustained manner. When released, growth factors stimulate differentiation of stem cells to form chondral or subchondral tissue accordingly. The latter is also stimulated by hydroxyapatite, which is the addition of regeneration construct layer II. After implantation and gradual degradation, regeneration construct withdraws from the defect site, making space for newly formed cartilage and subchondral tissue.

## Claims

1. A method of preparation of a construct for regeneration of articular joint cartilage, wherein said construct comprises a two-layer scaffold matrix, in which: a layer I comprises a scaffold of biodegradable polymers and natural additives; and a layer II comprises a scaffold of biodegradable polymer and natural additives; wherein the natural additives comprise cellulose, wherein said method comprises:
(a) preparation of polymer solutions for layers I and II;
(b) preparation of the regeneration construct layer I and II scaffolds by solution cryo-electrospinning;
(c) conversion of cellulose acetate to cellulose of the regeneration construct layer I and II scaffolds;
(d) surface modification of the regeneration construct layer I scaffold by treating with ozone;
(e) embedding of biologically active molecules in the regeneration construct layer I scaffold;
(f) sterilization of the regeneration construct layer I and II scaffolds;
(g) combining of the regeneration construct layer I and II scaffolds in order to form final regeneration construct.

2. The method of preparation of construct according to claim 1, which comprises additional drying steps following to (b), (d) and (e) steps.

3. The method of preparation of construct according to claim 1 or 2, in which the first layer and second layer polymer solutions are made of a biodegradable polymer selected from polycaprolactone, polylactic acid, and poly(lactic-co-glycolic acid).

4. The method of preparation of construct according to any of claims 1 - 3, in which biologically active molecules added to the solution of the first layer are growth factors.

5. The method of preparation of construct according to any of claims 1 - 4, in which solvents are polar organic solvents.

6. The method of preparation of construct according to any of claims 1 - 5, in which growth factors are incorporated to the biodegradable scaffold through functional groups.

7. The method of preparation of construct according to any of claims 1 - 6, in which growth factors stimulate differentiation of stem cells to form chondral or subchondral tissue of layer I and layer II accordingly.

## Patentansprüche

1. Verfahren zur Herstellung eines Konstrukts zur Regeneration von Gelenkknorpel, wobei das Konstrukt eine zweischichtige Gerüstmatrix umfasst, in der: eine Schicht I ein Gerüst aus biologisch abbaubaren Polymeren und natürlichen Additiven umfasst; und eine Schicht II ein Gerüst aus biologisch abbaubarem Polymer und natürlichen Additiven umfasst; wobei die natürlichen Additive Cellulose umfassen, wobei das Verfahren umfasst:
(a) Herstellung von Polymerlösungen für die Schichten I und II;
(b) Herstellung der Gerüste der Regenerationskonstrukt-Schichten I und II durch Kryoelektrospinnen der Lösung;
(c) Umwandlung von Celluloseacetat in Cellulose der Gerüste der Regenerationskonstrukt-Schichten I und II;
(d) Oberflächenmodifizierung des Gerüsts der Regenerationskonstrukt-Schicht I durch Behandlung mit Ozon;
(e) Einbettung biologisch aktiver Moleküle in das Gerüst der Regenerationskonstrukt-Schicht I;
(f) Sterilisation der Gerüststrukturen der Regenerationskonstrukt-Schicht I und II;
(g) Kombination der Gerüststrukturen der Regenerationskonstrukt-Schicht I und II, um das endgültige Regenerationskonstrukt zu bilden.

2. Verfahren zur Herstellung eines Konstrukts nach Anspruch 1, das zusätzliche Trocknungsschritte nach den Schritten (b), (d) und (e) umfasst.

3. Verfahren zur Herstellung eines Konstrukts nach Anspruch 1 oder 2, bei dem die Polymerlösungen der ersten und zweiten Schicht aus einem biologisch abbaubaren Polymer bestehen, das aus Polycaprolacton, Polymilchsäure und Poly(milchsäure-co-glycolsäure) ausgewählt ist.

4. Verfahren zur Herstellung eines Konstrukts nach einem der Ansprüche 1 bis 3, bei dem biologisch aktive Moleküle, die der Lösung der ersten Schicht hinzugefügt werden, Wachstumsfaktoren sind.

5. Verfahren zur Herstellung eines Konstrukts nach einem der Ansprüche 1 bis 4, bei dem Lösungsmittel polare organische Lösungsmittel sind.

6. Verfahren zur Herstellung eines Konstrukts nach einem der Ansprüche 1 bis 5, bei dem Wachstumsfaktoren durch funktionelle Gruppen in das biologisch abbaubare Gerüst eingebaut werden.

7. Verfahren zur Herstellung eines Konstrukts nach einem der Ansprüche 1 bis 6, bei dem Wachstumsfaktoren die Differenzierung von Stammzellen zur Bildung von chondralem oder subchondralem Gewebe der Schicht I und Schicht II entsprechend stimulieren.

## Revendications

1. Procédé de préparation d'une construction pour la régénération du cartilage articulaire, dans laquelle ladite construction comprend une matrice d'échafaudage à deux couches, dans laquelle : une couche I comprend un échafaudage de polymères biodégradables et d'additifs naturels ; et une couche II comprend un échafaudage de polymères biodégradables et d'additifs naturels ; dans laquelle les additifs naturels comprennent de la cellulose, dans laquelle ladite méthode comprend :
(a) la préparation de solutions de polymères pour les couches I et II ;
(b) la préparation des échafaudages de construction de régénération des couches I et Il par cryo-électrofilage de solution ;
(c) la conversion de l'acétate de cellulose en cellulose des échafaudages des couches I et II de la construction de régénération ;
(d) modification de la surface de l'échafaudage de la couche de construction de régénération I par traitement à l'ozone ;
(e) incorporation de molécules biologiquement actives dans l'échafaudage de la couche I de la construction de régénération ;
(f) stérilisation des échafaudages des couches I et II de la construction de régénération
(g) combinaison des échafaudages des couches I et II de la construction de régénération afin de former la construction de régénération finale.

2. Procédé de préparation de la construction selon la revendication 1, qui comprend des étapes de séchage supplémentaires après les étapes (b), (d) et (e).

3. Procédé de préparation d'une construction selon la revendication 1 ou 2, dans laquelle les solutions de polymère de la première couche et de la deuxième couche sont constituées d'un polymère biodégradable choisi parmi le polycaprolactone, l'acide polylactique et l'acide poly(lactic-co-glycolique).

4. Procédé de préparation d'une construction selon l'une des revendications 1 à 3, dans laquelle les molécules biologiquement actives ajoutées à la solution de la première couche sont des facteurs de croissance.

5. Procédé de préparation de la construction selon l'une des revendications 1 à 4, dans laquelle les solvants sont des solvants organiques polaires.

6. Procédé de préparation d'une construction selon l'une des revendications 1 à 5, dans laquelle les facteurs de croissance sont incorporés à l'échafaudage biodégradable par l'intermédiaire de groupes fonctionnels.

7. Procédé de préparation d'une construction selon l'une des revendications 1 à 6, dans laquelle les facteurs de croissance stimulent la différenciation des cellules souches pour former un tissu chondral ou sous-chondral de la couche I et de la couche Il en conséquence.
